# EUROPEAN PATENT APPLICATION

(11) **EP 2 711 708 A1**
(43) Date of publication of application: **26.03.2014**
(21) Application number: 12185337.8
(22) Date of filing: 21.09.2012
(51) Int. Cl.: G01N 33/68, G01N 33/50

(54) **Determination of tryptophan metabolites for assessing liver cell function**

(71) Applicant: Ruprecht-Karls-Universität Heidelberg, 69117 Heidelberg (DE)
(72) Inventor: The designation of the inventor has not yet been filed
(74) Representative: Dick, Alexander

(57) **Abstract**

The present invention relates to a method for assessing damage in cells expressing the tryptophan degradation pathway, comprising (a) determining the amount of tryptophan and /or the amount of at least one tryptophan degradation product in a sample comprising metabolites of said cells expressing the tryptophan degradation pathway, (b) comparing the amount determined in step (a) with a reference amount, (c) thereby determining damage in cells expressing the tryptophan degradation pathway. The present invention further relates to a method for identifying a compound detrimental to cells expressing the tryptophan degradation pathway, and to the use of the amount of tryptophan and / or the amount of at least one tryptophan degradation product in a sample from cells expressing the tryptophan degradation pathway for determining damage in cells expressing the tryptophan degradation pathway and / or for identifying a compound detrimental to cells expressing the tryptophan degradation pathway. The invention also related to a device for assessing damage in cells expressing the tryptophan degradation pathway, to a kit comprising instructions to carry out the method of the present invention, to a method for assessing liver damage and / or impairment of liver function in a subject, and to a method for assessing if treatment of liver damage and / or impairment of liver function is effective in a subject.

## Description

The present invention relates to a method for assessing damage in cells expressing the tryptophan degradation pathway, comprising (a) determining the amount of tryptophan and /or the amount of at least one tryptophan degradation product in a sample comprising metabolites of said cells expressing the tryptophan degradation pathway, (b) comparing the amount determined in step (a) with a reference amount, (c) thereby determining damage in cells expressing the tryptophan degradation pathway. The present invention further relates to a method for identifying a compound detrimental to cells expressing the tryptophan degradation pathway, and to the use of the amount of tryptophan and / or the amount of at least one tryptophan degradation product in a sample from cells expressing the tryptophan degradation pathway for determining damage in cells expressing the tryptophan degradation pathway and / or for identifying a compound detrimental to cells expressing the tryptophan degradation pathway. The invention is also related to a device for assessing damage in cells expressing the tryptophan degradation pathway, to a kit comprising instructions to carry out the method of the present invention, to a method for assessing liver damage and / or impairment of liver function in a subject, and to a method for assessing if treatment of liver damage and / or impairment of liver function is effective in a subject.

The liver is a vital organ for vertebrates fulfilling a wide range of functions important for the body. It plays a major role in metabolism and has a number of vital functions in the body, including glycogen storage, decomposition of red blood cells and synthesis of plasma proteins. The liver is also the largest gland in the human body. It lies below the diaphragm in the thoracic region of the abdomen. It produces bile acids, which aid in digestion and it regulates a wide variety of high-volume biochemical reactions. One of the most important functions of the liver is detoxification of various compounds that have entered the body with food, drinking, through the skin, via injection of medicaments (drugs), or by various other pathways. This is also the reason why the liver or cultivated liver cells are subjects of major interest when it comes to testing the toxicity of chemical compounds. Around 70% of cytoplasmaticc mass of the liver is made up by hepatocytes; hepatocytes are the cells most crucial for most of the functions described above, they are involved in protein synthesis, detoxification, protein storage, carbohydrate transformation, the synthesis of cholesterol, bile acids and phospholipids as well as the excretion of exogenous and endogenous substances. Hepatocytes also initiate the production and secrection of the bile.

Toxicity testing currently is performed either by contacting cultured cells or test animals with a compound and determining if any signs of toxicity can be detected. However, the use of animals in toxicity testing is expensive and has been questioned for ethical reasons. Testing compounds on cultured cells has been increasingly useful, especially since cultured cells mimicking liver function, like cultured primary hepatocytes and HepaRG cells, a cell line mimicking most of the metabolic functions of primary human hepatocytes, have become available. While the maintenance of freshly isolated primary hepatocytes in an ex vivo culture is possible within limitations, (sub)-culturing of these cells is impossible at this time. (Kim HM, Han SB, Hyun BH, Ahn CJ, Cha YN, Jeong KS, Oh GT. Functional human hepatocytes: isolation from small liver biopsy samples and primary cultivation with liver-specific functions. J Toxicol Sci. 1995 Nov;20(5):565-78; Vinken M, Vanhaecke T, Rogiers V. Primary hepatocyte cultures as in vitro tools for toxicity testing: quo vadis? Toxicol In Vitro. 2012 Apr;26(3):541-4.) Thus primary hepatocytes have to be reisolated from patient samples frequently, limiting availability and comparability between donors, as well as making them expensive to obtain. While it is possible to cultivated HepaRG cells in cell culture following a complicated scheme involving a minimum of 4 weeks of differentiation, cultivation and maintenance of function is labor intensive (Gripon P, Rumin S, Urban S, Le Seyec J, Glaise D, Cannie I, Guyomard C, Lucas J, Trepo C, Guguen-Guillouzo C. Infection of a human hepatoma cell line by hepatitis B virus. Proc Natl Acad Sci U S A. 2002 Nov 26;99(24):15655-60.) On the other hand, tests on these cells typically involve end-point determinations leading to the destruction of the cells. Thus, methods minimizing the consumption of these cells would be desirable.

There is, thus, a need in the art for means and methods to comply with the aforementioned needs.

Accordingly, the present invention relates to a method for assessing damage in cells expressing the tryptophan degradation pathway, comprising (a) determining the amount of tryptophan and / or the amount of at least one tryptophan degradation product in a sample comprising metabolites of said cells expressing the tryptophan degradation pathway, (b) comparing the amount determined in step (a) with a reference amount, (c) thereby determining damage in cells expressing the tryptophan degradation pathway.

The methods of the present invention, preferably, are in vitro methods. Moreover, they may comprise steps in addition to those explicitly mentioned. For example, further steps of the method for assessing damage in cells expressing the tryptophan degradation pathway may relate, e.g., to preparation steps for the sample of step a), or a normalization calculation in step b). Moreover, one or more of said steps may be performed by automated equipment.

As used herein, the term "assessing" relates to detecting or diagnosing if damage has occurred or is occurring to the cells of the present invention.

The term "damage", as used herein, relates to a, preferably detrimental, deviation from normal of the condition of a cell. The damage may be persistent or temporary. Preferably, the damage is caused by external compounds interacting with the cell. Preferably, the damage is a distortion or a loss of membrane integrity, distortion or loss of metabolic capabilities, inhibition of mitochondrial function, induction of apoptosis or loss of differentiation. Preferably, the damage leads to a change in the tryptophan metabolism of the cell, more preferably, the change is detectable by one of the methods of the present invention. It is to be understood that damage does not necessarily relate to damage of isolated cells. Thus, preferably, the term "damage" may also relate to damage of a tissue or of an organ, like, e.g. liver. Such damage may be caused by chemical compounds like, e.g. toxins like alcohols or other solvents, by biological agents, like, e.g. viruses (e.g. Hepatitis virus A, B, or C), or by other conditions like, e.g. oxygen deprivation or cancer. It is to be understood that also diseases associated with damage to cells expressing the tryptophan degradation pathway, like, preferably, liver cirrhosis, hepatitis, cancer, preferably hepatocellular cancer, or metastatic cancers of other organs e.g colorectal carcinoma, autoimmune diseases (e.g. Multiple Sclerosis, Lupus), acute/chronic inflammation, depression, neurodegenerative diseases (Alzheimer, Parkinson's disease, Huntington's disease, Amyotrophic lateral sclerosis (ALS)), aging, intoxination with certain poisons (e.g. metabolic endproducts, microbiological exo- and / or endo - toxins, food additives, contaminants such as insecticides or pesticides, drugs and metabolic intermediates of drugs, or alcohol), haemochromatosis, Wilson's disease, primary sclerosing cholangitis, primary biliary cirrhosis, Budd-Chiari, Gilberts's Syndrome, Glycogen storage disease type II, antitrypsin deficiency, alagille syndrome, progressive familial intrahepatic cholestasis as well as other metabolic diseases are encompassed by damage as referred to by the present invention. Thus, the method for determining damage in cells expressing the tryptophan degradation pathway is, preferably, a method for diagnosing disease causing damage to liver cells. More preferably, the method for determining damage in cells expressing the tryptophan degradation pathway is a method for diagnosing liver cirrhosis, hepatitis, cancer, preferably hepatocellular cancer on the tryptophan degradation pathway.

As used herein, the term "tryptophan degradation pathway" relates to the catabolic enzymatic conversions initiating or mediating the removal of tryptophan from metabolism. Preferably, said enzymatic conversions comprise formylkynurenine and / or kynurenine as intermediates.

The term "cell expressing the tryptophan degradation pathway" as used herein, relates to any cell expressing at least the enzymes catalyzing the catabolism of the amino acid tryptophan to N-formylkynurenine. Preferably, the cell is a cell expressing the complete tryptophan degradation pathway as described e.g. in Moffett JR, Namboodiri MA. "Tryptophan and the immune response." Immunol Cell Biol 2003;81:247-65; Schröcksnadel K, et al., "Monitoring tryptophan metabolism in chronic immune activation." Clin Chim Acta. 2006 Feb;364(1-2):82-90; Hirata et al., "indoleamine 2,3-dioxygenase. note i. catalytic and molecular properties." Acta Vitaminol Enzymol, 1975. 29(1-6): p. 288-90; Frumento et al., "tryptophan-derived catabolites are responsible for inhibition of t and natural killer cell proliferation induced by indoleamine 2,3-dioxygenase." J Exp Med, 2002. 196(4): p. 459-68; Takikawa et al., "mechanism of interferon-gamma action. characterization of indoleamine 2,3-dioxygenase in cultured human cells induced by interferon-gamma and evaluation of the enzyme-mediated tryptophan degradation in its anticellular activity." J Biol Chem, 1988. 263(4): p. 2041-8; Guillemin et al., "kynurenine pathway metabolism in human astrocytes." Adv Exp Med Biol, 1999. 467: p. 125-31; Munn et al., "prevention of allogeneic fetal rejection by tryptophan catabolism." Science, 1998. 281(5380): p. 1191-3; Munn et al., "inhibition of t-cell proliferation by macrophage tryptophan catabolism." J Exp Med, 1999. 189(9): p. 1363-72; Pfefferkorn, "interferon gamma blocks the growth of toxoplasma gondii in human fibroblasts by inducing the host cells to degrade tryptophan." Proc Natl Acad Sci USA, 1984. 81(3): p. 908; Uyttenhove et al., "evidence for a tumoral immune resistance mechanism based on tryptophan degradation by indoleamine 2,3-dioxygenase." Nat Med, 2003. 9(10): p. 1269-74; or in LÖB et al.,. "inhibitors of indoleamine-2,3-dioxygenase for cancer therapy: can we see the wood for the trees?" Nat Rev Cancer. 2009 jun;9(6):445-52.

More preferably, the cell is a, preferably, cultured, primary hepatocyte, most preferably the cell is a HepaRG cell (Moffett JR, Namboodiri MA., loc. cit.; Watanabe et al., "stereospecificity of hepatic 1-tryptophan 2,3-dioxygenase." Biochem J, 1980. 189(3): p. 393-405; Knox and Mehler, "the adaptive increase of the tryptophan peroxidase-oxidase system of liver." Science, 1951. 113(2931): p. 237-8; Liao et al., "impaired dexamethasone-mediated induction of tryptophan 2,3-dioxygenase in heme-deficient rat hepatocytes: translational control by a hepatic eif2alpha kinase, the heme-regulated inhibitor". J Pharmacol Exp Ther. 2007 dec;323(3):979-89). Preferably, the cell is a metazoan cell (e.g. an insect cell), more preferably a mammalian cell e.g., preferably, a mouse or a rat cell, most preferably a human cell (Braidy et al. "Changes in kynurenine pathway metabolism in the brain, liver and kidney of aged female Wistar rats." FEBS J. 2011 Nov;278(22):4425-34; Ohira et al., "Expression of tryptophan 2,3-dioxygenase in mature granule cells of the adult mouse dentate gyrus." Mol Brain. 2010 Sep 5;3(1):26; Kanai et al., "Tryptophan 2,3-dioxygenase is a key modulator of physiological neurogenesis and anxiety-related behavior in mice." Mol Brain. 2009 Mar 27;2:8.) It is, however, understood that the cell can be a bacterial, an archeal, or a, preferably unicellular, eukaryotic cell.

The term "tryptophan" or "trp" relates to the amino acid known under that term (IUPY AC name: 2-Amino-3-(1H-indol-3-yl)propanoic acid). Preferably, the term relates to the proteinogenic amino acid L-tryptophan (IUPAC name: (2S)-2-amino-3-(1H-indol-3-yl)propanoic acid). As used herein, the term "tryptophan degradation product" relates to any detectable intermediate of tryptophan catabolism such as L-Formylkynurenine ((S)-2-amino-4-(2-formamidophenyl)-4-oxobutanoic acid), Kynurenine ((S)-2-amino-4-(2-aminophenyl)-4-oxobutanoic acid), Kynurenic Acid (4-hydroxyquinoline-2-carboxylic acid) ,3-Hydroxykynurenine ((S)-2-amino-4-(2-amino-3-hydroxyphenyl)-4-oxobutanoic acid), Anthranilic Acid (2-aminobenzoic acid), 3-Hydroxyanthranilic Acid (2-amino-3-hydroxybenzoic acid), Quinolinic Acid (pyridine-2,3-dicarboxylic acid), Picolinic Acid, 5-Hydroxytryptophan ((S)-2-amino-3-(5-hydroxy-1H-indol-3-yl)propanoic acid), but not intermediates such as , ATP, NADPH, AcetylCoA, 2-Amino-muconic-semialdehyde, 2-Amino-3.Carboxy-muconic-semialdehyde. involving the tryptophan degradation pathway as specified. Preferably, the tryptophan degradation product is kynurenine. The term "kynurenine", as used herein relates to the compound with the IUPAC designation 2-Amino-4-(2-aminophenyl)- 4-oxo-butanoic acid, preferably, the term relates to L-kynurenine (or (S)-kynurenine), i.e. (S)-2-Amino-4-(2-aminophenyl)- 4-oxo-butanoic acid.

The term "determining" relates to the quantification of the amount of tryptophan and / or the amount of at least one tryptophan degradation product present in a sample, i.e. measuring the amount or concentration of said tryptophan and / or tryptophan degradation product, preferably semi-quantitatively or quantitatively. Measuring can be done directly or indirectly. The determining of the amount of tryptophan and / or of a tryptophan degradation can be accomplished in a variety of ways known to the skilled person, e.g., preferably, by chromatographic methods, more preferably, by an enzymatic assay. The amount or concentration of tryptophan is, most preferably, determined with a method comprising incubation of said sample with a polypeptide comprising tryptophanase (L-tryptophan indole-lyase (deaminating; pyruvate-forming), EC 4.1.99.1) activity (Burns and DeMoss, "Properties of tryptophanase from Escherichia coli." Biochim. Biophys. Acta 65 (1962) 233-244; Cowell, et al., "Tryptophanase from Aeromonas liquifaciens. Purification, molecular weight and some chemical, catalytic and immunological properties." Biochim. Biophys. Acta 315 (1973) 449-46; Newton et al., "Properties of crystalline tryptophanase." J. Biol. Chem. 240 (1965) 1211-1218)

In accordance with the present invention, determining the amount of tryptophan and / or tryptophan degradation product can be achieved by all known means for determining the amount said tryptophan and / or tryptophan degradation product in a sample, provided that they are adapted to specifically detect the tryptophan and / or tryptophan degradation product of the present invention. Said methods comprise, preferably, biosensors, optical devices coupled to enzymatic assays, biochips, analytical devices such as mass-spectrometers, NMR-analyzers, or chromatography devices. Moreover, the signal strength can, preferably, be correlated directly or indirectly (e.g. reverse-proportional) to the amount of tryptophan and /or tryptophan degradation product present in a sample. Preferably, the amount of tryptophan and / or tryptophan degradation product is specified by one of the methods specified herein below in the examples.

The term "amount" as used herein encompasses the absolute amount of the tryptophan and /or tryptophan degradation product referred to herein, the relative amount or concentration of the tryptophan and / or tryptophan degradation product referred to herein as well as any value or parameter which correlates thereto. Such values or parameters comprise intensity signal values from all specific physical or chemical properties obtained from the tryptophan and / or tryptophan degradation product referred to herein by measurements. It is to be understood that values correlating to the aforementioned amounts or parameters can also be obtained by all standard mathematical operations.

The term "sample", as used herein, refers to any sample comprising metabolites of the cell, tissue, or organ of the present invention. Preferably, the sample is a sample of a body fluid, a sample of separated cells, a sample from a tissue or an organ, or a sample of wash/rinse fluid obtained from an outer or inner body surface. More preferably, samples are body fluids like blood, plasma, serum, urine, saliva, lacrimal fluid, or stool. Most preferably, the sample is a sample of cell culture supernatant from cells of the present invention. Samples can be obtained by well known techniques and include, preferably, scrapes, swabs or biopsies. Such samples can be obtained by use of brushes, (cotton) swabs, spatula, rinse/wash fluids, punch biopsy devices, puncture of cavities with needles or surgical instrumentation. Preferably, samples originate from the urogenital tract, the anal canal, the oral cavity, the upper aerodigestive tract and the epidermis. More preferably, samples originate from blood or liver. Separated cells and/or cell-free liquids may be obtained from cell culture supernatants, body fluids, or the tissues or organs by separating techniques such as filtration, centrifugation, or cell sorting. Separated cells, preferably, are lysed before being used as samples in the present invention by one of the methods well known to the skilled artisan. It is to be understood that the sample may be further processed in order to carry out the method of the present invention. Particularly, amino acids might be extracted and/or purified from the obtained sample by methods and means known in the art. Thus, the term sample also may relate to amino acids and mixtures of low-molecular weight compounds purified and/or extracted from any sample as mentioned above.

"Comparing" as used herein encompasses comparing the amount of the tryptophan and / or tryptophan degradation product referred to herein which are comprised by the sample to be analyzed with an amount of the tryptophan and / or tryptophan degradation product in a suitable reference sample as specified elsewhere herein. It is to be understood that comparing as used herein refers to a comparison of corresponding parameters or values, e.g., an absolute amount of the tryptophan and / or tryptophan degradation product as referred to herein is compared to an absolute reference amount of said tryptophan and / or tryptophan degradation product; a concentration of the tryptophan and / or tryptophan degradation product as referred to herein is compared to a reference concentration of said tryptophan and / or tryptophan degradation product; an intensity signal obtained from the tryptophan and / or tryptophan degradation product as referred to herein in a test sample is compared to the same type of intensity signal of said tryptophan and / or tryptophan degradation product in a reference sample. The comparison referred to in the methods of the present invention may be carried out manually or computer assisted. For a computer assisted comparison, the value of the determined amount or ratio may be compared to values corresponding to suitable reference values which are stored in a database by a computer program. The computer program may further evaluate the result of the comparison by means of an expert system. Accordingly, the result of the identification referred to herein may be automatically provided in a suitable output format.

The terms "reference value" or "reference amount" as used herein refer to an amount of tryptophan and / or tryptophan degradation product, which allows assessing if damage is to be assumed for the cell, tissue, organ, or subject the sample is derived from. A suitable reference value may be determined from a reference sample to be analyzed together, i.e. simultaneously or subsequently, with the sample.

Preferably, the reference amount as used herein is derived from samples for which it is known if the cells the sample is derived from were damaged or not. This reference amount level may be a discrete figure or may be a range of figures. Evidently, the reference level or amount may vary between individual chemical compounds, i.e. tryptophan or tryptophan degradation product, determined. The measuring system therefore, preferably, is calibrated with a sample or with a series of samples comprising known amounts of said chemical compound. It is understood by the skilled person that in such case the amount of tryptophan or tryptophan degradation product will preferably be expressed as arbitrary units (AU). Thus, preferably, the amounts of tryptophan or tryptophan degradation product is determined by comparing the signal obtained from the sample to signals comprised in a calibration curve.

The reference amount applicable for an individual subject may vary depending on various parameters such as type of cell or tissue, incubation conditions, or, in the case of samples from subjects, age, gender, or subpopulation. Thus, a suitable reference amount may be determined by the methods of the present invention from a reference sample to be analyzed together, i.e. simultaneously or subsequently, with the test sample. Moreover, a threshold amount can be preferably used as a reference amount. Preferably, an amount of tryptophan or tryptophan degradation product as detailed herein in the examples is used.

The most common measure of correlation is the Pearson Product Moment Correlation (called Pearson's correlation for short). The Pearson product-moment correlation coefficient (or Pearson correlation coefficient for short) is a measure of the strength of a linear association between two variables and is denoted by r.

The Pearson correlation coefficient, r, can take a range of values from +1 to -1. A value of 0 indicates that there is no association between the two variables. A value greater than 0 indicates a positive association, that is, as the value of one variable increases so does the value of the other variable. A value less than 0 indicates a negative association, that is, as the value of one variable increases the value of the other variable decreases.

The stronger the association of the two variables the closer the Pearson correlation coefficient, r, will be to either +1 or -1 depending on whether the relationship is positive or negative, respectively. Achieving a value of +1 or -1 means that all your data points are included on the line of best fit - there are no data points that show any variation away from this line. Values for r between +1 and -1 (for example, r = 0.8 or -0.4) indicate that there is variation around the line of best fit. The closer the value of r to 0 the greater the variation around the line of best fit.

Reference amounts can, in principle, be calculated for a group of samples as specified herein based on the average or median values for a given tryptophan and / or tryptophan degradation product by applying standard methods of statistics. In particular, accuracy of a test such as a method aiming to assess an event, or not, is best described by its receiver-operating characteristics (ROC) (see especially Zweig 1993, Clin. Chem. 39:561-577). The ROC graph is a plot of all of the sensitivity versus specificity pairs resulting from continuously varying the decision threshold over the entire range of data observed. The performance of an assessment method depends on its accuracy, i.e. its ability to correctly allocate samples to a certain state. The ROC plot indicates the overlap between the two distributions by plotting the sensitivity versus 1-specificity for the complete range of thresholds suitable for making a distinction. On the y-axis is sensitivity, or the true-positive fraction, which is defined as the ratio of number of true-positive test results to the sum of number of true-positive and number of false-negative test results. This has also been referred to as positivity in the presence of a condition. It is calculated solely from the subgroup comprising samples from damaged cells. On the x-axis is the false-positive fraction, or 1-specificity, which is defined as the ratio of number of false-positive results to the sum of number of true-negative and number of false-positive results. It is an index of specificity and is calculated entirely from the subgroup of samples from undamaged cells. Because the true- and false-positive fractions are calculated entirely separately, by using the test results from two different subgroups, the ROC plot is independent of the prevalence of the condition in the cohort. Each point on the ROC plot represents a sensitivity/-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis or prediction for a given event with a proper balance of sensitivity and specificity, respectively. Accordingly, the reference to be used for the methods of the present invention can be generated, preferably, by establishing a ROC for said cohort as described above and deriving a threshold amount there from. Dependent on a desired sensitivity and specificity for a diagnostic method, the ROC plot allows deriving suitable thresholds. Preferably, the reference amounts lie within the range of values that represent a sensitivity of at least 75% and a specificity of at least 45%, or a sensitivity of at least 80% and a specificity of at least 40%, or a sensitivity of at least 85% and a specificity of at least 33%, or a sensitivity of at least 90% and a specificity of at least 25%.

Advantageously, it was found in the work underlying the present invention, that cultured cells expressing the tryptophan degradation pathway, e.g. hepatocytes, degrade tryptophan from the surrounding medium at a detectable rate and that these cells secrete detectable amounts of kynurenine into the medium. Surprisingly, it was further found that the detection of metabolites of tryptophan degradation allows for a sensitive, non-destructive determination of viability of said cells. This allows for sensitive testing for compounds detrimental to the viability of said cells, without a need for destroying said cells in the analysis.

The definitions made above apply mutatis mutandis to the following:

The present invention further relates to a method for identifying a compound detrimental to cells expressing the tryptophan degradation pathway, comprising (a) contacting cells expressing the tryptophan degradation pathway with a compound suspected to be detrimental to cells expressing the tryptophan degradation pathway, (b) determining damage in cells expressing the tryptophan degradation pathway according to the method of any one of claims 1 to 7, (c) identifying a compound detrimental to cells expressing the tryptophan degradation pathway based on the result of the determination of step b).

The term "compound", as used herein, refers to a chemical molecule, i.e. any organic or inorganic substance. The organic molecule may belong to any known chemical class of molecules. Preferably, organic molecules are lipids, fatty acids, purines, pyrimidines, alkaloids, amino acids, peptides, polypeptides, proteins, biogenic amines, isoprenoids or steroids. The term "compound suspected to be detrimental" to cells expressing the tryptophan degradation pathway relates to any compound for which it is known, suspected, or cannot be excluded that it causes, mediates, or otherwise inflicts damage to said cells. The term "compound detrimental" to cells expressing the tryptophan degradation pathway, accordingly, relates to a compound identified to cause, mediate, or otherwise inflict damage to said cells. The skilled person knows that the amount of damage inflicted by a compound typically depends on several factors, like, e.g., concentration of the compound, duration of its interaction with said cells, presence or absence of other compounds, temperature, and the like. Thus, the skilled person knows that the assertion that a compound is identified as being detrimental to cells expressing the tryptophan degradation pathway will typically only be valid under the provisio of the specific conditions under which the compound was tested in the method of the present invention. Also, there are several legal and administrative prescriptions, as well as directions in the state of the art, for example by the European REACH Initiative (http://ec.europa.eu/enterprise/sectors/chemicals/reach/index_en.htm) or ADME toxicity testing of new drugs (Balani et al. (2005), Curr Top Med Chem. 5(11):1033-8). Strategy of utilizing in vitro and in vivo ADME tools for lead optimization and drug candidate selection.) indicating under which conditions a compound suspected to be detrimental has to be tested for damage it may inflict, thus the skilled person knows how to adjust conditions accordingly to obtain meaningful results.

The term "contacting" as used in the context of the method of the present invention is understood by the skilled person. Preferably, the term relates to bringing a compound of the present invention into physical contact with a cell of the present invention and thereby allowing the compound and the cell to interact.

The present invention also relates to the use of the amount of tryptophan and / or the amount of at least one tryptophan degradation product in a sample from cells expressing the tryptophan degradation pathway for determining damage in cells expressing the tryptophan degradation pathway and / or for identifying a compound detrimental to cells expressing the tryptophan degradation pathway.

The present invention further relates to a device for determining damage in cells expressing the tryptophan degradation pathway, comprising an analyzing unit for determining the amount of tryptophan and / or the amount of at least one tryptophan degradation product in a sample from cells expressing the tryptophan degradation pathway and an evaluation unit having tangibly embedded an algorithm for carrying out a comparison of the amount determined by the analyzing unit with a reference.

The term "device" as used herein relates to a system of means comprising at least the aforementioned means operatively linked to each other as to allow the determination. Preferred means for determining damage in cells expressing the tryptophan degradation pathway and means for carrying out the comparison are disclosed above in connection with the methods of the invention and herein below in the examples. How to link the means in an operating manner will depend on the type of means included into the device. For example, where means for automatically determining the amount of tryptophan and / or the amount of at least one tryptophan degradation product are applied, the data obtained by said automatically operating means can be processed by, e.g., a computer program in order to establish a result (i.e. identifying that damage in cells expressing the tryptophan degradation pathway has occurred). Preferably, the means are comprised by a single device in such a case. Said device may accordingly include an analyzing unit for the measurement of the amount of tryptophan and / or the amount of at least one tryptophan degradation product in a sample and an evaluation unit for processing the resulting data for the diagnosis. Alternatively, where means such as test stripes are used for determining the amount of tryptophan and / or the amount of at least one tryptophan degradation product, the means for establishing a result may comprise control stripes or tables allocating the determined amount to an amount known to be associated with damage or with no damage to the cells of the invention. Preferred means for detection are disclosed in connection with embodiments relating to the methods of the invention above. The means may appear as separate devices in such an embodiment and are, preferably, packaged together as a kit. The person skilled in the art will realize how to link the means without further inventive skills. Preferred devices are those which can be applied without the particular knowledge of a specialized clinician, e.g., test stripes or electronic devices which merely require loading with a sample. The results may be given as output of parametric raw data, preferably, as absolute or relative amounts. It is to be understood that these data may need interpretation. However, also envisaged are expert system devices wherein the output comprises processed raw data, the interpretation of which does not require a specialized operator. Further preferred devices comprise the analyzing units/devices (e.g., HPLC/detector devices, spectrometers, biosensors, solid supports coupled to ligands specifically recognizing the analyte(s), Plasmon surface resonance devices, NMR spectrometers, mass-spectrometers etc.) or evaluation units/devices referred to above in accordance with the methods of the invention. Preferably, the evaluation unit comprises a data processor which is capable of generating an output file containing a diagnosis established based on the said comparison.

The present invention also relates to a kit comprising instructions to carry out a method of the present invention, a detection agent for the amount of tryptophan and / or the amount of at least one tryptophan degradation product in a sample, and standards for reference amounts allowing determining damage in the cell expressing the tryptophan degradation pathway of the present invention.

The term "kit" as used herein refers to a collection of the aforementioned components, preferably, provided separately or within a single container. The container, also preferably, comprises instructions for carrying out the method of the present invention. Examples for such components of the kit as well as methods for their use have been given in this specification. The kit, preferably, contains the aforementioned components in a ready-to-use formulation. Preferably, the kit may additionally comprise instructions, e.g., a user's manual for interpreting the results of any determination(s) with respect to the diagnoses provided by the methods of the present invention. Particularly, such manual may include information for allocating the amounts of of tryptophan and / or the amount of at least one tryptophan degradation product to the kind of result or diagnosis. Details are to be found elsewhere in this specification. Additionally, such user's manual may provide instructions about correctly using the components of the kit for determining the amount(s) of the respective biomarker. A user's manual may be provided in paper or electronic form, e.g., stored on CD or CD ROM. The present invention also relates to the use of said kit in any of the methods according to the present invention.

Further, the present invention relates to a method for determining organ damage and / or impairment of organ function in a subject, comprising determining the amount of tryptophan and / or the amount of at least one tryptophan degradation product in a sample of said subj ect, thereby determining organ damage and / or impairment of organ function in a subject.

The term "organ", as used herein, relates to a differentiated part of the body performing one or more specific function(s) and comprising or consisting of cells expressing the tryptophan degradation pathway. Preferably, the organ is a distinct grouping of tissue into a distinct structure. More preferably, the organ is skin or nerve tissue, e.g. brain. Most preferably, the organ is liver. It is, however also envisaged by the present invention that the organ is the immune system or a structural, e.g. a lymph node, bone marrow, spleen, or the like, or a functional, e.g. the T-cell-, the NK cell, or the B-cell compartment, part thereof.

As detailed herein above, the term "damage" in the method for assessing damage in cells expressing the tryptophan degradation pathway may also relate to damage of a tissue or of an organ, like, e.g. liver. Thus, "determining organ damage", as used herein, relates to determining damage to cells expressing the tryptophan degradation pathway, preferably liver cells, which is the result or the cause of organ damage, with the method for assessing damage in cells expressing the tryptophan degradation pathway. Likewise, the term "determining impairment of organ function" relates to determining damage to cells expressing the tryptophan degradation pathway, preferably liver cells, which is the result or the cause of an impaired organ function, with the method for assessing damage in cells expressing the tryptophan degradation pathway.

The term "subject", as used herein, relates to a vertebrate, preferably a mammal, more preferably a cat, dog, hamster, guinea pig, horse, sheep, cattle, pig, rat, mouse, rabbit, monkey, or goat. Most preferably, the subject is a human. Preferably, the subject is suspected or known to be afflicted with organ damage and / or impairment of organ function and / or any ongoing inflammatory event. Symptoms of the aforementioned conditions are well known in the art and include jaundice, hepatic failure, sleep disorders and portal-systemic encephalopathy in patients with liver cirrhosis, chronic hepatitis C, acute hepatic failure, Hepatic encephalopathy, hepatic coma (Moroniet al., "Increase in the content of quinolinic acid in cerebrospinal fluid and frontal cortex of patients with hepatic failure." J Neurochem 1986; 47 (6): 1667; Basile et al., "The relationship between plasma and brain quinolinic acid levels and the severity of hepatic encephalopathy in animal models of fulminant hepatic failure." J Neurochem 1995; 64 (6): 2607; Wiltfang et al., "Sleep disorders and portal-systemic encephalopathy following transjugular intrahepatic portosystemic stent shunt in patients with liver cirrhosis. Relation to plasma tryptophan." Adv Exp Med Biol. 1999;467:169-76; Loock et al., "Amino acid dysbalance in liver failure is favourably influenced by recirculating albumin dialysis (MARS)." Liver. 2002;22 Suppl 2:35-9; Comai et al., "Effects of PEG-interferon alpha plus ribavirin on tryptophan metabolism in patients with chronic hepatitis C." Pharmacol Res. 2011 Jan;63(1):85-92. Epub 2010 Oct 19; Albrecht and Jones, "Hepatic encephalopathy: molecular mechanisms underlying the clinical syndrome." J Neurol Sci. 1999 Nov 30;170(2):138-46; Bergqvist et al., "Plasma and brain levels of oxindole in experimental chronic hepatic encephalopathy: effects of systemic ammonium acetate and L-tryptophan." Pharmacol Toxicol. 1999 Sep;85(3):138-43; Herneth et al., "Role of tryptophan in the elevated serotonin-turnover in hepatic encephalopathy." J Neural Transm. 1998;105(8-9):975-86; Carpenedo et al., "Oxindole, a sedative tryptophan metabolite, accumulates in blood and brain of rats with acute hepatic failure." J Neurochem. 1998 May;70(5):1998-2003; Zoli et al., "Binding of tryptophan to albumin in liver cirrhosis: a reappraisal of the problem." Hepatogastroenterology. 1981 Apr;28(2):87-9; Delorme et al., "Relationship between plasma and brain tryptophan in pigs during experimental hepatic coma before and after hemodialysis with selective membranes." J Neurochem. 1981 Mar;36(3):1058-66; Ono et al., "Tryptophan and hepatic coma." Gastroenterology. 1978 Feb;74(2 Pt 1):196-200; Altman and Greengard, "Tryptophan pyrrolase induced in human liver by hydrocortisone: effect on excretion of kynurenine." Science. 1966 Jan 21;151(3708):332-3).

The present invention relates to a method for determining if treatment of organ damage and /or impairment of organ function and / or treatment of an ongoing inflammatory event and / or treatment of autoimmune disease is effective in a subject undergoing said treatment, comprising: a) determining the amount of tryptophan and / or the amount of at least one tryptophan degradation product in a first sample from said subject, b) determining the amount of tryptophan and / or the amount of at least one tryptophan degradation product in a second sample from said subject, wherein said second sample is taken after said first sample, c) comparing the amount of tryptophan and / or the amount of at least one tryptophan degradation product from step a) to the amount of tryptophan and / or the amount of at least one tryptophan degradation product determined in step b), thereby determining if treatment of organ damage and / or impairment of organ function and / or treatment of an ongoing inflammatory event and / or treatment of autoimmune disease is effective.

As used herein, the term "treatment" refers to all measures employed for ameliorating the diseases or disorders referred to herein or the symptoms accompanied therewith. Said treating as used herein also includes an entire restoration of the health with respect to the diseases or disorders referred to herein. It is to be understood that treating as used in accordance with the present invention may not be effective in all subjects to be treated.

The term "inflammatory event" relates to any kind of inflammation, preferably an inflammatory event is an acute or chronic inflammation. Preferably the inflammatory event involves an adaptive immune response and / or an innate immune response. Preferably, the inflammatory event occurs subsequent to a viral or bacterial infection, is an anti-tumor response and / or follows an organ transplantation. More preferably, the inflammatory event involves tissue damage. Most preferably, the inflammatory event is severe inflammatory bowl disease, sepsis, inflammatory arthritis, inflammatory dermatitis, or systemic inflammatory response syndrome.

The term "autoimmune disease" is known to the skilled person and relates to an immune response directed against an antigen within the body of the host. Preferably, the autoimmune disease is any kind of disease or illness causally linked to the immune response being directed to a self-antigen i.e. any disease caused by the failure of a subject's immune system to recognize its own constituent parts as self. Preferably, the autoimmune disease of the present invention is innate or acquired. More preferably, the acquired autoimmune disease is induced by a foreign antigen or by an autochthonous antigen. Preferably, the autoimmune disease involves both T-cell and B-cell responses; it is, however, also envisaged by the present invention that the autoimmune disease involves T-cell responses or B-cell responses. More preferably, the autoimmune disease is Multiple Sclerosis, Lupus, Hashimotos thyroiditis, Hashimoto's encephalopathy, Graves Disease, Rheumatoid Arthritis, acute disseminated encephalomyelitis, autoimmune hepatitis, autoimmune pancreatitis, autoimmune uveitis, Crohn's disease, Goodpasture's syndrome, gastrointestinal pemphigoid, Polymyositis, ulcerative colitis, Vasculitis, or Wegener's granulomatosis.

The term "first sample" relates to a sample obtained from a subject at a point in time before the "second sample" of the present invention. Preferably, the first sample is obtained at least one day, at least two days, at least three days, at least four days, at least five days, at least six days, at least one week, at least two weeks, at least three weeks, at least one month, at least two months, at least three months, at least four months, at least five months, at least six months, at least one year, at least two years, at least three years, at least four years, or at least five years before the second sample. It is to be understood that the terms first sample and second sample are only used to indicate a chronological relationship between two given samples, meaning that a first sample with respect to a certain second sample can be the second sample with respect to a third sample. E.g., if the sequence of samples taken was A, then B, then C, this means that sample B is the second sample in relation to sample A, but a first sample in relation to sample C.

All references cited in this specification are herewith incorporated by reference with respect to their entire disclosure content and the disclosure content specifically mentioned in this specification.

### Figure Legends

**Fig. 1****: Determination of Tryptophan levels in HepaRG cells (****Fig. 1A****) and Primary Human hepatocytes (****Fig. 1B****) over time as a marker for cell viability.** For comparison, cells not expressing the Tryptophan degradation pathway have been analyzed for Tryptophan degradation (Fig. 1C).
**Fig. 2****: Example of a EIC filter setup for the analysis of different Tryptophan metabolites using Mass-Spectrometry assisted RP-HPLC.**
   10µl of a 10µM Standard solution of Tryptophan as well as 4 different Tryptophan metabolites (Kynurenine, 3-OH-Kynurenine, Anthranilic Acid, 3-OH-Anthranilic Acid) has been injected into the System. Peaks of the respective metabolites can be easily identified and integrated to allow the generation of a standard curve based on the area under curve (not shown). On the X-Axis the time of the run is plotted, Peaks appearing at a given time are confirmed to be the right metabolite peak by confirming the right mass in a coupled Mass Spectrometer and displayed in the diagram.

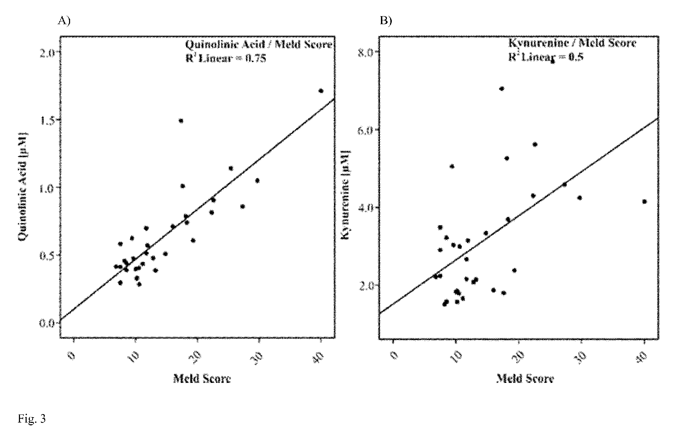

**Fig. 3****: Correlation of Tryptophan metabolites (Kyn andQuinA) with liver dysfunction in patients suffering from primary hepatocellular carcinoma.**
   The Model for End-Stage Liver Disease (MELD) score is used as a prognostic measure for hepatic insufficiency/failure. The tryptophan metabolites (Kyn and QuinA) were all found to correlate with the established clinical indices MELD score. In detail, the MELD score positively correlated with serum levels of QuinA (r=0.84) (Fig. 3A)and Kyn(r=0.54) (Fig. 3B) (p<0.0001 for both investigations). Linear correlation coefficient has been determined (r²).

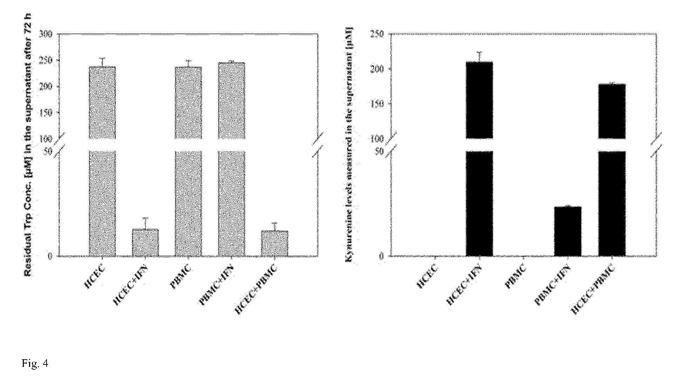

**Fig. 4****: Determination of Tryptophan and accumulation of Kynurenine by immune cells (endothelium and blood cells) as marker for an ongoing inflammation.**
   Human Cornea endothelial Cells (HCEC) and Peripheral Blood Mononuclear Cell (PBMC)were cultivated for 72 h either alone, in the presence of IFN-g (a classical pro-inflammatory cytokine. Also, an allogeneic response was set by coculture of HCEC with allo PBMC. After 72 h cell supernatants were harvested and Tryptophan and Tryptophan Metabolite (Kyn) were analyzed as described in Example A and B

The following Examples shall merely illustrate the invention. They shall not be construed, whatsoever, to limit the scope of the invention.

### Example 1: Measurement of Tryptophan using Apotryptophanase

Tryptophan content of a given sample can be measured using an enzymatic assay as described below. Sample material, e,g cell culture supernatants or patient samples have to be collected by a skilled person, Cell culture supernatants have to be treated by the addition of an appropriate solvent to remove residual proteins from the sample. Possible solvents include, but are not limited to, e.g. organic solvents such as ethanol or acetone, but also acids such as trifluoronic acid or perchloric acid. The appropriate solvent for the exact purpose can be selected by the skilled person. For this example protein removal is achieved by the addition of 10% (v/v) 2,4M perchloric acid to the cell culture supernatant. Alternatively, when semi-automated RP-HPLC shall be used for sample analysis (Example 2), Protein removal should be performed by the addition of 10% (v/v) concentrated Trifluoroacetic acid to the sample. After addition of the solvent, the sample has to be incubated at room temperature for five minutes after which the denaturated proteins are pelleted by a centrifugation step (8000xg / 15 minutes at 4°C) and discarded.

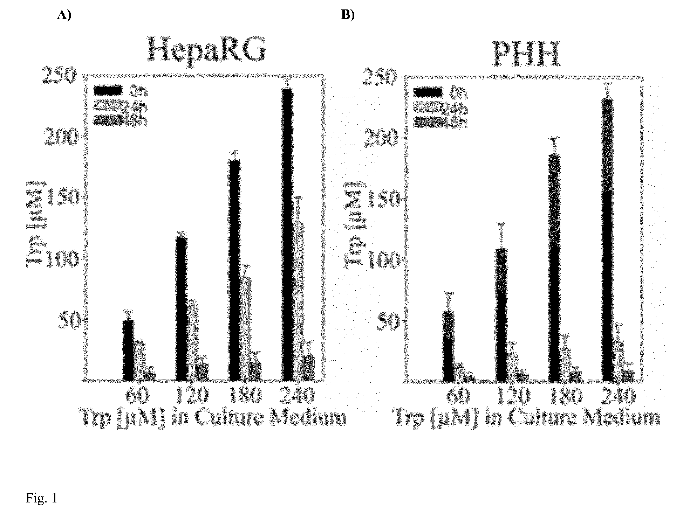

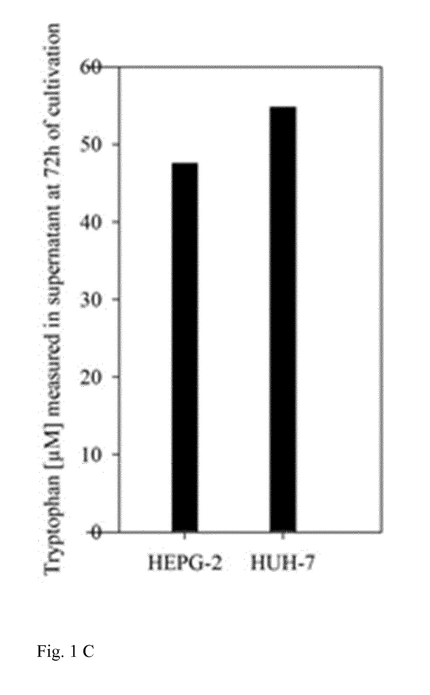

The sample is then neutralized by the addition of 6N Sodiumhydroxide at a ratio of 2:1 (v/v) to the amount of perchloric acid used. This step is not needed when RP-HPLC is used for tryptophan determination (Example B). Sample pH has to be adjusted to pH 7.8 by a skilled person. 100µl of the protein free solution containing Tryptophan is to be diluted 4:1 in a Buffer containing 6.7 mM Ammoniumsulfate, 0.2 mM Pyridoxal-5-Phosphate, 0.2 M Potassiumphosphate, 2 mM Dithithreitole as well as 10 IU Apotryptophanase. The resulting solution is then mixed carefully without vortexing. The solution is mixed with 250 µl Cyclohexane and incubated for 30-60 minutes on a rocking shaker at 37°C. If very high concentrations of Tryptophan are contained in the sample (higher than 200 µM) appropriate dilutions of the sample might be necessary, alternatively the amount of Apotryptophanase has to be increased accordingly. Following incubation, the reaction is stopped by the addition of 150 µl of a 3 M solution of Sodiumhydroxide and a second round of incubation for 30 minutes on a rocking shaker is performed. After incubation the sample is stored for 10 minutes at room temperature without shaking to allow the separation of the cyclohexane containing the reaction product of Apotryphanase (Indole) from the aqueous solution,. 75 µl of Cyclohexane are removed from the sample and measured in a photometer at 279 nm extinction. Emission is measured at 306 nm. The amount of Tryptophane contained in the sample can be derived from the generation of a standard curve by titrating appropriate amounts of Indole into Cyclohexane and measuring of the Emission of the solution in a photometer at the wavelengths give above. An example of this Technique for the measurements of cell culture supernatants is give in Fig. 1.

### Example 2: Measurement of Tryptophan metabolites using RP-HPLC connected to positive mode Mass spectrometry.

Semi-automated metabolite analysis can be perfomed using an Agilent 1200 Series RP-HPLC equipped with an automated sample loader and a C-18-S column, directly coupled to a Thermo-Scientific- EXACT Orbitrap Mass Spectrometer set to measure in positive mode. 0.05% (v/v) Trifluoroacetic acid in ultra-pure Acetonitrile is used as an Eluent. System calibration and setup is known to the skilled artisan.

Samples are prepared before measurement as described in Example a). If necessary dilutions to the sample should be done by the addition of ultra-pure HPLC grade water to avoid contaminations.

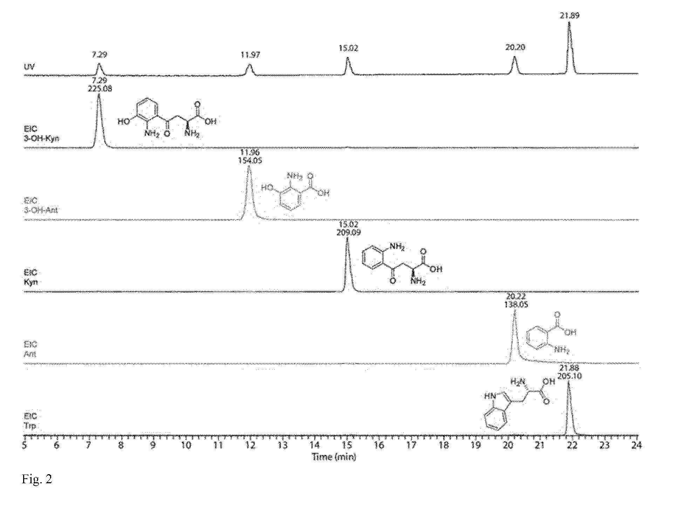

Standard calibration mixtures are prepared from pure, analytical grade tryptophan degradation products in defined concentrations. Appropriate concentration range has to be determined depending on the samples tested, which the skilled artisan will be able to do. At least 3 different concentrations of the calibration mixtures have to be measured in triplex prior to the measurement of any unknown sample allowing the generation of a standard curve for each analyte. Additionally at least one blank measurement should be performed in duplex to allow subtraction of background signals. Appropriate masses of the tryptophan degradation products can be taken from literature, note; for measurement in positive mode the mass of one proton should be added to the absolute mass of the degradation products. Extracted Ion Count (EIC) filters can be setup by the skilled Artisan in the software supplied with the mass spectrometer to allow easy identification of the correct mass peaks reflecting the individual masses of the respective tryptophan degradation products. An example of such an EIC filter for the analysis of Kynurenine, Tryptophan, 3-OH-Kynurenine, Anthranilic Acid and 3-OH-Anthranilic Acid is given in Figure 3. Other EIC filters for different metabolites can be easily added by the skilled Artisan. Sample are loaded onto the C-18-S column and eluted using a linear gradient of acetonitrile from 0-22.5% over a runtime of 25 minutes at 21°C. Mass peaks are recorded automatically and the resulting mass spectra is subjected to analysis on the EIC filter (Fig. 3). An Example of the Mass-Spectrometry-based measurement of Tryptophan metabolits in Patient samples is provided in Fig. 2

### Example 3: Measurement of Trp degradation Metabolites as markers for Inflammation.

Human Cornea epithelia cells (HCEC) were cultivated either in the presence or absence of Interferon-gamma or in the presence or absence of allogeneic stimulated Plasma Monocyte Derived Cells (PBMC) as a model system for an ongoing inflammation. Supernatants of the cultivated HCECs were taken at respective time points and analyzed using the methods described in Example 1 or 2. Correlations between Inflammatory activity and Trp Metabolites could be identified.

## Claims

1. A method for assessing damage in cells expressing the tryptophan degradation pathway, comprising
(a) determining the amount of tryptophan and / or the amount of at least one tryptophan degradation product in a sample comprising metabolites of said cells expressing the tryptophan degradation pathway,
(b) comparing the amount determined in step (a) with a reference amount,
(c) thereby determining damage in cells expressing the tryptophan degradation pathway.

2. The method of claim 1, wherein the cells are cultured primary hepatocytes.

3. The method of claim 1 or 2, wherein the cells are HepaRG cells.

4. The method of any one of claims 1 to 3, wherein the sample is a cell-free sample comprising metabolites of said cells expressing the tryptophan degradation pathway.

5. The method of any one of claims 1 to 4, wherein the amount of tryptophan and / or the amount of at least one tryptophan degradation product is determined by a chromatographic method.

6. The method of any one of claims 1 to 5, wherein the amount of tryptophan and / or the amount of at least one tryptophan degradation product is determined by an enzymatic assay.

7. The method of any one of claims 1 to 6, wherein the amount of tryptophan is determined with a method comprising incubation of said sample with a polypeptide comprising tryptophanase (L-tryptophan indole-lyase (deaminating; pyruvate-forming), EC 4.1.99.1) activity.

8. A method for identifying a compound detrimental to cells expressing the tryptophan degradation pathway, comprising
(a) contacting cells expressing the tryptophan degradation pathway with a compound suspected to be detrimental to cells expressing the tryptophan degradation pathway,
(b) determining damage in cells expressing the tryptophan degradation pathway according to the method of any one of claims 1 to 7,
(c) identifying a compound detrimental to cells expressing the tryptophan degradation pathway based on the result of the determination of step b).

9. Use of the amount of tryptophan and / or the amount of at least one tryptophan degradation product in a sample from cells expressing the tryptophan degradation pathway for determining damage in cells expressing the tryptophan degradation pathway and / or for identifying a compound detrimental to cells expressing the tryptophan degradation pathway.

10. A device for assessing damage in cells expressing the tryptophan degradation pathway, comprising an analyzing unit for determining the amount of tryptophan and / or the amount of at least one tryptophan degradation product in a sample from cells expressing the tryptophan degradation pathway and an evaluation unit having tangibly embedded an algorithm for carrying out a comparison of the amount determined by the analyzing unit with a reference.

11. The device of claim 10, wherein the evaluation unit comprises a data processor which is capable of generating an output file containing a diagnosis established based on the said comparison.

12. A kit comprising instructions to carry out the method of the present invention, a detection agent for the amount of tryptophan and / or the amount of at least one tryptophan degradation product in a sample, and standards for reference amounts allowing determining damage in liver cells.

13. A method for assessing organ damage and / or impairment of organ function in a subject, comprising determining the amount of tryptophan and / or the amount of at least one tryptophan degradation product in a sample of said subject, thereby determining organ damage and / or impairment of organ function in a subject.

14. The method of claim 13, wherein the organ damage is hepatocellular cancer.

15. A method for assessing if treatment of organ damage and / or impairment of organ function is effective in a subject undergoing said treatment, comprising:
a) determining the amount of tryptophan and / or the amount of at least one tryptophan degradation product in a first sample from said subject,
b) determining the amount of tryptophan and / or the amount of at least one tryptophan degradation product in a second sample from said subject, wherein said second sample is taken after said first sample,
c) comparing the amount of tryptophan and / or the amount of at least one tryptophan degradation product from step a) to the amount of tryptophan and / or the amount of at least one tryptophan degradation product determined in step b),
thereby determining if treatment of organ damage and / or impairment of organ function is effective.
